# EUROPEAN PATENT APPLICATION

(11) **EP 3 170 491 A1**
(43) Date of publication of application: **24.05.2017**
(21) Application number: 14897556.8
(22) Date of filing: 23.09.2014
(51) Int. Cl.: A61K 9/10, A61K 31/4164, A61K 38/05, A61K 47/50, A61P 1/00

(54) **POLYCOMPLEX GEL FOR TREATING DISEASES OF INTESTINAL/DIGESTIVE TRACT**

(30) Priority: 15.07.2014 RU 2014128856
(71) Applicant: Obschestvo S Ogranichennoi Otvetstvennostyu "Gelizovit", Moscow 115035 (RU)
(72) Inventor: KHOROVETS, Genrikh Markovich, Moscow 115583 (RU)
(74) Representative: Bucher, Ralf Christian
(86) International application number: PCT/RU2014/000708
(87) International publication number: WO 2016/010451

(57) **Abstract**

The invention relates to the chemical and pharmaceutical industry and consists of a rectal gel substance for treating diseases of the intestinal/digestive tract, including nonspecific ulcerative colitis, Crohn's disease, fistulae, etc., and also complications during the course of treatment of said diseases. The invention provides an antimicrobial and antiprotozoal effect and has good penetration capabilities. The technical result achieved by carrying out the claimed invention consists in increasing the effectiveness of treating non-infectious inflammatory diseases of the intestinal/digestive tract, and in shortening the duration of treatment. Said technical result is achieved by means of a polycomplex gel for treating diseases of the intestinal/digestive tract, said gel containing metronidazole and thymogen as active substances, and containing the following auxiliary substances, in the following composition and ratio of components, in grams, with a total mass of gel of 150 grams: metronidazole: 0.1-2g; thymogen: 0.001-0.05g; carbopol: 0.2-1.0g; propylene glycol: 8.0-11.0g; EDTA disodium salt: 0.001-0.08g; propyl parahydroxybenzoate: 0.015-0.04g; methyl parahydroxybenzoate: 0.035-0.08g; sodium hydroxide: an amount sufficient for producing the necessary pH; distilled water: the remainder.

## Description

### Field of the Invention

The present invention relates to the chemical and pharmaceutical industry and consists of a rectal gel substance for treating diseases of the intestinal/digestive tract, including nonspecific ulcerative colitis, Crohn's disease, fistulas, etc., as well as complications arising in the course of said diseases.

The invention provides for an antimicrobial and antiprotozoal effect and good penetration capabilities.

### Background of the Invention

Noninfectious inflammatory diseases of the intestinal/digestive tract, such as ulcerative colitis and Crohn's disease have been one of the most serious problems in modem gastroenterology. Morbidity with ulcerative colitis and with Crohn's disease is growing every year, and it is mainly amongst the adult population, which makes them socially significant diseases.

Similarity of these affections of the intestinal/digestive tract is due to the fact that they are the basis of the mucosa chronic destruction; the course of these diseases has a phase character with periods of exacerbation and stages of remission. Up to now, a real etiopathogenetic cause of both inflammatory processes rise and the transition thereof to a chronic form is not elucidated. At the same time, these diseases significantly differ as to the macroscopic and histologic picture as well as to the location thereof in the digestive tract.

In the case of ulcerative colitis, inflammation covers exclusively the large intestine mucosa, while spreading successively from the distal part to the proximal part thereof, and embracing, in heavy cases, the entire large bowel, including rectum.

In the case of Crohn's disease, the inflammatory process can affect any parts from mouth to anus. Most often for this disease, a combined injury of lower parts of the small intestine and of the large bowel, with a segmented localization of injuries when an affected part has neighboring parts with an unaltered mucosa.

To treat non-infectious inflammatory intestinal diseases, use is made of various preparations, in particular, it is known the sulphasalazine preparation (Encyclopedia of drugs. Register of medicines of Russia, RLS, Moscow, OOO RLS-2005, 12th edition, p. 826-827) providing an antibacterial and anti-inflammatory effect. In the intestinal connective tissue, it decomposes to 5-aminosalycilic acid (5-ASA) determining the sulphasalazine anti-inflammatory properties, and to sulphapyridine that is a concurrent antagonizer of p-aminobenzoic acid terminating synthesis of foliates in the cells of microorganisms and determining the antibacterial activity. The drawbacks of said preparation in the treatment of ulcerative colitis and of Crohn's disease consist in a strong dependence from the condition of intestinal normoflora that takes part in the sulphasalazine biotransformation and in the release of the active component 5-ASA, when sulphapyridine, the second component of the preparation practically brakes and inhibits the preparation specific activity. It may drastically increase allergic manifestations, dyskinesia disorders and induce liver function disturbances.

Otherwise, it is known the preparation salophalc representing encapsulated tablets, the capsules of which are dissolvable in the intestine, and that contain mesalazine, representing 5-aminosalicylic acid (Encyclopedia of drugs. Register of medicines of Russia, RLS, Moscow, OOO RLS-2005, 12th edition, p.780-781). This preparation represents an anti-inflammatory substance, it inhibits synthesis of arachidonic acid metabolites, the activity of neutrophil lipoxygenase, it retards migration, degranulation and phagocytosis of neutrophils, secretion of immunoglobulins by lymphocytes, it binds and destroys oxygen free radicals. It is indicated to treat ulcerative colitis and Crohn's disease. As disadvantages of the preparation, one can mention, together with marked allergic manifestations, functional derangements of intestine, of nervous, cardiovascular and urinary systems, that are declared as side-effects. It results in recommendations to use the preparation only under medical supervision and with regular analyses of blood and urine.

### Summary of the Invention

It is an object of the present invention to provide a preparation to treat inflammatory diseases of the intestinal/digestive tract, free of the above mentioned disadvantages and providing antimicrobial, locally anesthetizing and anti-inflammatory capabilities, that does not induce an active cutaneous anaphylaxis or does not produces any irritating effect onto the rectum mucosa.

The technical result achieved by carrying out the claimed invention consists in increasing the effectiveness of treating non-infectious inflammatory diseases of the intestine and in shortening the duration of treatment with no side effects or complications, that is achieved thanks to a combined approach to form a set of components and is corroborated by examination records of patients that have followed a course of treatment with said preparations, said examinations being performed by independent specialists. Another technical result consists in providing a combined effect of the preparation both onto the intestine mucosa and the submucous layer, which provides for a stable curative effect and for conditions to prevent recidivation.

Said technical result is achieved thanks to a polycomplex gel for treating diseases of the intestinal/digestive tract, said gel containing metronidazole and thymogen as active substances, and containing auxiliary substances, in the following composition and ratio of components, in grams, with a total mass of gel of 150 g:
metronidazole: 0.1 to 2.0 g;
thymogen: 0.001 to 0.05 g;
carbopol: 0.2 to 1.0 g;
propylene glycol: 8.0 to 11.00 g;
EDTA disodium salt: 0.001 to 0.08 g;
propyl parahydroxybenzoate: 0.015 to 0.04 g;
methyl parahydroxybenzoate: 0.035 to 0.08 g;
sodium hydroxide: an amount sufficient for providing the necessary pH;
distilled water: the remainder.

### Pharmacologic effect of active substances:

Metronidazole provides an antimicrobial and antiprotozoal effect. The mechanism of action thereof consists in the penetration of the preparation nitro group into the respiratory chain of protozoa and anaerobes. As a result, the respiration processes in microorganisms are disturbed, which contributes to the death of cells. What is more, in some species of anaerobic microorganisms, this preparation disturbs DNA synthesis which favors the degradation thereof. Metronidazole is active against: protozoa (Trichomonas vaginalis, Lamblia, Entamoeba histolytica, Leishmania0, aerobic gram-negative microorganisms (bacteroids Bacteroides fragilis, B. caccae, B. uniformis, B. fragilis, B. ovatus, B. thetaiotaomicron, B. distasonis, B. Vulgatus, fusobcteria Prevotella P. buccae, P. bivia, P. Disiens), anaerobic gram-negative bacilli (Clostridium, Eubacterium), anaerobic gram-positive cocci (Peptococcus spp., Peptostreptococcus spp).

Thymogen provides for an immunomodifying effect and represents a dipeptide influencing the reactions of cellular, humoral immunity and the non-specific resistance of the organism. It stimulates regeneration processes in the cases of oppression thereof, improves the course of the cellular metabolism processes. It intensifies processes of lymphoid cells differentiation, provides the possibility to stimulate the colony-forming activity of bone marrow cells, induces expression of differentiation receptors on lymphocytes, normalizes the number of t-helpers, T-suppressors and the ratio thereof in patients having different immunodeficiency conditions.

The selection of auxiliary substances is conditioned by requirements to the gel as a whole, which finally resulted in the following choice:
1. Carbopol that is used as a gel modifier for providing a dynamic consistency point and plastic viscosity.
2. Propylene glycol is added to the gel composition for improving solvability of methyl parahydroxybenzoate and propyl parahydroxybenzoate .
3. EDTA disodium salt (trilon B) is added to the gel composition due to the possibility to find, in the utilized components, traces of variable valence metals inducing oxidation processes.
4. Methyl parahydroxybenzoate and propyl parahydroxybenzoate have been used as preservatives to prevent formation of unwanted microflora.

The main advantage of the claimed polycomplex gel is the efficiency thereof on the background of absence of side effects or complications, which is achieved thanks to a combined approach to the formation of a set of components and corroborated by examination records of patients that have followed a course of treatment with said preparations, said examinations being performed by independent specialists. It is advisable as well to separately point out a combined effect of the claimed polycomplex gel both on the intestine mucosa and on the subcutaneous layer, which favors the achievement of a stable curative effect together with the conditions to prevent recidivation. An important element to note is that the non-specific ulcerative colitis represents an autoimmune disease, while the presence of thymogen in the gel composition inhibits the development of autoimmune local reactions. Thus, the vicious pathologic circle is broken, and conditions for normalizing the intestine wall are created. This effect is intensified by the presence of metronidazole in the gel composition, which enables to restore injured structures both of the mucosa and the subcutaneous layer, while providing an antimicrobial and antiprotozoal effect. The mechanism of action thereof consists in the penetration of the preparation nitro group into the respiratory chain of protozoa and anaerobes. The interaction of metronidazole and thymogen provides for a wide combined curative effect, normalizes ionic, acid-base and salt balance, while stabilizing the cellular membrane and inactivating cytokines and inflammatory mediators. On the background of the above described, the main advantage of the claimed polycomplex gel consists in its combined effect on all the large intestine hemostasis systems. Thanks to the combination of the gel components, the tissue metabolism and microcirculation are normalized and, as a result, the disease is eliminated, while the metronidazole/thymogen combination enables both the treatment of the main disease and the prevention of complications. Unfortunately, existing preparations do not provide such an effect.

The polycomplex gel for treating diseases of the intestinal/digestive tract is ranged in the class of substances regulating the interstitial metabolism of substances on the background of intracellular metabolism, while stabilizing the cellular membrane and reducing inflammatory reaction manifestations together with lowering the concentration of anti-inflammatory cytokines, prostaglandins and other mediators of inflammation in tissues. The mechanism of the preparation curative effect is bound to the stabilization of the microcirculatory bed in cellular membranes, to the improvement of rheological properties in the capillary network of the large intestine mucous membrane and to the inhibition of the primary autoimmune inflammatory reaction in the intestine wall, which finally results in the interruption of pathological processes in the large intestine wall, in the normalization of the metabolism in tissues and to a reverse development of pathological processes.

And what is more, the preparations components complement each other, when thymogen complements the curative effect of metronidazole, and the combination of these two active substances produces a pronounced local anti-inflammatory and reparative effect.

Thanks to innovative technologies that have enabled to create the present preparation, the following properties have been achieved that are not accessible for existing modem preparations:
- the ability to completely recover a normal condition of the large intestine and of the anus;
- a high security thanks to a minimal toxicity and to physiological feedback technologies.

Besides, it is necessary to point out that the claimed polycomplex gel is characterized by the absence of allergenic properties. Considering the fact that nowadays, an obligatory stage of a new medicine study is the evaluation of the allergenicity thereof, the claimed polycomplex gel has passed a verification on the basis of the Arthus-Sakharov test. For this purpose, six rabbits of both sexes weighting from 2.0 to 2.5 kg were injected with thymogen under sterile conditions subcutaneously on one side of the back with the dose of 10 mg/kg in 1.0 ml of sodium chloride isotonic solution and 1 ml of hors serum subcutaneously into the back on the other side. The rabbits of the control group were injected, instead of thymogen, with 1 ml of sodium chloride isotonic solution. The injections of thymogen and of horse serum were repeated for 6 times with a 5-6 days' interval. In the place of horse serum injection in rabbits of both groups, an equal local reaction was observed as hyperemia, hemorrhages and intumescence. In the place of injections of thymogen and of isotonic sodium chloride solution, no reactions were found. A prolonged intravenous injection of thymogen in three courses of 5 days each one with 7 days' intervals was not accompanied by any titer growth of allergic agglutinating autoantibodies.

Thus, an investigation of the polycomplex gel with standard methods showed that thymogen was not allergenic. What is more, subsequent clinical observations showed that thymogen demonstrates determined desensitizing properties, which allowed the application thereof in a combined treatment of atopic conditions.

The presence of rectal thymogen in the gel composition and the desensitizing properties thereof have a determined hypoallergenicityof the polycomplex gel, which was confirmed by the gel allergenicity studies that were carried out in accordance with the methodical recommendations of FGU NCESMP ("Manual of preclinical studies of medicines", part 1, Moscow, 2012).

The experiments used 30 white-sided guinea pigs of both sexes weighting 250 to 300 g.

Active cutaneous anaphylaxis. Active cutaneous anaphylaxis was studied in agreement with the "Methodical recommendations for evaluation of allergenic properties of medicines" on white-sided guinea pigs of both sexes weighting 250-300 g. Sensitization of the animals was carried out by rectal administration of the preparation for 21 days. The polycomplex gel dosage is determined on the basis of a therapeutic dose of active substance (metronidazole) for humans (100 mg or 1.43 mg/kg), that is why for guinea pigs, the therapeutic dose calculated with the coefficient of species recalculation equal to 4.7 is as high as 6.7 mg/kg. For said sensibilization, 2 dosages were used: a therapeutic dose of 6.7 mg/kg and a double dose of 13.7 mg/kg.

The experiment used 30 guinea pigs. The animals were divided into 3 groups of 10 animals each (5♂ and 5♀) according to the random sorting method:
1^{st} group: control (gel placebo administration);
2^{nd} group: polycomplex gel of 6.7 mg/kg;
3^{rd} group: polycomplex gel of 13.4 mg/kg.

For detecting sensibilization, use was made of the studied polycomplex gel as a subcutaneous injection in the amount of 50 µl Preliminary experiments on intact guinea pigs determined that the subcutaneous injection of the preparation under study did not induce irritations.
14 days after the last sensibilization procedure, some hair was clipped on the guinea pigs and then 50 µl of studied polycomplex gel were injected subcutaneously. To check the skin reactivity, the same animal was injected subcutaneously with 50 µl of placebo gel at another place.
30 minutes after the preparation injection, all the investigated and control animals were injected intravenously with 0.5 ml of 1% Evans dye solution.
30 minutes after that, the animals were killed and the size of colored spots at the places of injection of polycomplex gel and of placebo gel. The experimental results are given in a table.

**Table 25**

| Reaction of active cutaneous anaphylaxis in guinea pigs (males and females) after rectal administration of the polycomplex gel under study* | | | | |
|---|---|---|---|---|
| Preparation, dose injected to guinea pigs for sensibilization | Blue spot diameter (mm) at the place of injection: | | | |
| | for the preparation | | for 0.9% sodium chloride solution | |
| | M | SD | M | SD |
| Placebo | 3.5 | 0.55 | 3.7 | 0.63 |

| Investigated polycomplex gel | | | | |
|---|---|---|---|---|
| 6.7 mg/kg | 3.8 | 0.75 | 3.4 | 1.0 |
| 13.4 mg/kg | 4.1 | 0.65 | 3.9 | 0.45 |

| | | | | |
|---|---|---|---|---|
| * No certain differences found between the groups of laboratory animals. | | | | |

As shown in the table, the sizes of colored spots on the internal surface of the skin, at the place of the final injection in animals submitted to the sensibilization procedure did not exceed those of the control group animals. The data obtained testify that the polycomplex gel under study does not induce any reaction of active cutaneous anaphylaxis in guinea pigs. Thus, the investigation of the allergization activity of the polycomplex gel carried out by means of the active cutaneous anaphylaxis evaluation on guinea pigs showed that the preparation under study does not demonstrate any allergization capabilities.

The polycomplex gel for treating a nonspecific ulcerative colitis was submitted to the evaluation of the local irritation effect that was carried out in the course of the subchronic toxicity study on rabbits. Since the preparation under study is presented under the medicine dosage form "rectal gel", i.e. designed for rectal administration, the evaluation of the local irritation effect was carried out on the basis of the rectum mucosa condition. For this purpose, after killing and the examination of necrotic tissues, a visual inspection of the rectum wall mucosa was carried out with a binocular lens in order to detect petechiae (small-spot hemorrhages) and mucosa ulcerous injuries. It was shown that, both in the control groups (placebo) of animals and in the groups of animals that were administered with the preparation under study, the rectum mucosa was without edema, hemorrhages or ulcerations.

Besides, histological studies were carried out. The mucous membrane, muscular layer and serous membrane are well visible on the mounts of the rectum in all the groups. A typical image of the mucosa infiltration with leucocytes was observed. The solitary follicles were without modifications. The muscular and serous membranes of the rectum had a normal structure.

The study of the local irritation effect showed that the administration of the claimed rectal polycomplex gel for 30 days had no irritating effect on the rectum mucosa.

Acute and subchronic toxicity, aftereffects and local irritation effect, as well as allergization capabilities and specific activity of the polycomplex gel for treating a non-specific ulcerative colitis were studied and an evaluation thereof was carried out. In rectal administration of the preparation under study with all the dosages, no lethal cases were recorded. No certain modifications were observed in the relative mass of the internal organs, in particular: the liver, kidneys, adrenal glands, spleen, thymus, stomach, pancreas, heart, lungs, brain. No other visible signs of intoxication of any kind were recorded. The body volume increment and the temperature of animals receiving the preparation in all the three dosages did not differ from the similar indices of control animals that received placebo.

It was shown that rectal administration of the preparation for 30 days in all the three dosages did not result in any statistically significant differences in such biochemical parameters as total protein, glucose, urea, creatinine content, activity of enzymes Ala-AT, AsT and alkaline phosphatase, hemoglobin concentration, hematocrit, erythrocyte, leukocyte, reticulocyte and thrombocyte content, and parameters of the differential blood count.

A study of the investigated preparation aftereffect carried out two weeks after the end of the 30 days' rectal administration period showed that all the investigated parameters of animals that had received the preparation under study in dosages of 23, 115 and 230 mg/kg did not differ from the respective indices of the placebo group animals 2 weeks after the end of the administration course.

Preliminary results of the postmortem examination showed that the experiments to determine subchronic toxicity did not manifest morphological modifications in the liver, kidneys, adrenal glands, spleen, thymus, stomach, pancreas, heart, lungs, ovaries, testes and in the brain tissues under the effect of the investigated preparation.

A comparative histological analysis performed showed that no cytomorphological modifications were developed under the effect of the investigated preparation in the studied internal organs or tissues.

A study of the local irritation effect carried out on rabbits of both sexes showed that the administration of polycomplex gel for treating non-specific ulcerative colitis for 30 days did not have any irritating effect on the rectum mucous membrane.

A preliminary investigation of allergization capabilities of the polycomplex gel for treating nonspecific ulcerative colitis showed that the preparation under study did not demonstrate any allergization effect.

A study of the local anesthetic effect established that the preparation under study did not show any local irritating effect on the rabbit eye cornea.

It was shown that the polycomplex gel for treating non-specific ulcerative colitis provided a significant anti-inflammatory effect.

On the basis of the evaluation results of the acute and subchronic toxicity, aftereffects and local irritation effect, as well as of allergization capabilities and specific pharmacological activity (antimicrobial and anti-inflammatory) of the preparation under study, i.e. the "polycomplex gel for treating nonspecific ulcerative colitis", these results being obtained by biochemical, hematologic and histologic methods of investigation, this preparation can be recommended to treat nonspecific ulcerative colitis, Crohn's disease and fistulas as complications arising in the course of said diseases as well as of other intestinal diseases.

### Embodiments of the Invention

The polycomplex gel for treating nonspecific ulcerative colitis is administered rectally, daily (in the evening) once a day. The treatment course is determined depending on the disease gravity.

### Example 1.

For treating nonspecific ulcerative colitis, use can be made of the polycomplex gel having the composition as follows:
metronidazole: 1.0 g;
thymogen: 0.01 g;
carbopol: 1.0 g;
propylene glycol: 10.0 g;
EDTA disodium salt: 0.05 g;
propyl parahydroxybenzoate: 0.02 g;
methyl parahydroxybenzoate: 0.04 g;
sodium hydroxide: an amount sufficient for providing the necessary pH;
distilled water: the remainder.

### Example 2.

For treating Crohn's disease, use can be made of the polycomplex gel having the composition as follows:
metronidazole: 0.8 g;
thymogen: 0.002 g;
carbopol: 1.0 g;
propylene glycol: 10 g;
EDTA disodium salt: 0.04 g;
propyl parahydroxybenzoate: 0.025 g;
methyl parahydroxybenzoate: 0.04 g;
sodium hydroxide: an amount sufficient for providing the necessary pH;
distilled water: the remainder.

### Example 3.

For treating fistulas, use can be made of the polycomplex gel having the composition as follows:
metronidazole: 1.2 g;
thymogen: 0.015 g;
carbopol: 1.0 g;
propylene glycol: 10 g;
EDTA disodium salt: 0.05 g;
propyl parahydroxybenzoate: 0.02 g;
methyl parahydroxybenzoate: 0.04 g;
sodium hydroxide: an amount sufficient for providing the necessary pH;
distilled water: the remainder.

## Claims

1. polycomplex gel for treating diseases of the intestinal/digestive tract, **characterized in that** it comprises metronidazole and thymogen as active substances, and auxiliary substances, in the following composition and ratio of components, in grams, with a total mass of gel of 150 g:
metronidazole: 0.1 to 2.0 g;
thymogen: 0.001 to 0.05 g;
carbopol: 0.2 to 1.0 g;
propylene glycol: 8.0 to 11.00 g;
EDTA disodium salt: 0.001 to 0.08 g;
propyl parahydroxybenzoate: 0.015 to 0.04 g;
methyl parahydroxybenzoate: 0.035 to 0.08 g;
sodium hydroxide: an amount sufficient for providing the necessary pH;
distilled water: the remainder.
